(19) **Europäisches Patentamt**
**European Patent Office**
**Office européen des brevets**

(11) **EP 4 048 058 B1**

(12) **EUROPEAN PATENT SPECIFICATION**

(45) Date of publication and mention
of the grant of the patent:
**29.11.2023   Bulletin 2023/48**

(21) Application number: **20772081.4**

(22) Date of filing: **22.09.2020**

(51) International Patent Classification (IPC):
**A01G 7/00** *(2006.01)*        **G01N 27/04** *(2006.01)*
**G01N 33/00** *(2006.01)*

(52) Cooperative Patent Classification (CPC):
**A01G 7/00; G01N 27/026; G01N 33/0098**

(86) International application number:
**PCT/EP2020/076367**

(87) International publication number:
**WO 2021/078453 (29.04.2021 Gazette 2021/17)**

(54) **METHOD FOR DETERMINING A RELATIVE CHANGE IN A SAP FLOW DENSITY IN A VASCULAR PLANT, SOFTWARE PROGRAM, AND MEASUREMENT ARRANGEMENT**

VERFAHREN ZUR BESTIMMUNG EINER RELATIVEN VERÄNDERUNG DER SAFTFLUSSDICHTE IN EINER GEFÄSSPFLANZE, SOFTWAREPROGRAMM UND MESSANORDNUNG

PROCÉDÉ POUR DÉTERMINER UN CHANGEMENT RELATIF DE LA DENSITÉ DE FLUX DE SÈVE DANS UNE PLANTE VASCULAIRE, PROGRAMME LOGICIEL ET AGENCEMENT DE MESURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2019   EP 19204567**

(43) Date of publication of application:
**31.08.2022   Bulletin 2022/35**

(73) Proprietor: **Technische Universität München**
**80333 München (DE)**

(72) Inventors:
• **BRISCHWEIN, Martin**
**81371 München Bayern (DE)**

• **KÜBLER, Julius**
**85591 Vaterstetten Bayern (DE)**
• **FODERÁ, Giancarlo**
**80807 München Bayern (DE)**

(74) Representative: **K & H Bonapat**
**Patentanwälte Koch · von Behren & Partner mbB**
**Donnersbergerstraße 22A**
**80634 München (DE)**

(56) References cited:
**EP-A1- 3 104 135        WO-A1-2017/123010**
**KR-B1- 101 240 398**

**Description**

The invention relates to a method for determining a relative change in a sap flow density in a vascular plant, a software program realizing the method, and a measurement arrangement for determining the relative change in the sap flow density in the vascular plant.

WO 2017/123010 A1, KR 101 240 398 B1 and EP 3 104 135 A1 disclose relevant background art.

Sap flow occurs longitudinally along a sprout axis in vascular tissues of a plant and consists of a downward, i.e. towards the root, transport of assimilation products in the so-called phloem tissue, which can be found in the bark area, and an upward, i.e. towards the leaves, transport of water and ions, i.e. of an electrolyte, in the so-called xylem tissue which can be found in the wood area. There is a growth layer, also called cambium, between the phloem and the xylem, which is responsible for the thickness growth of the sprout. The sap flow is dominated by the share of the xylem. Its vascular tissue consists of dead, elongated cells which are connected longitudinally by screen walls and together form an elongated capillary system.

The xylem sap flow, i.e. sap flow in the xylem, is driven by a pressure or water potential difference between the root space and the atmosphere. Transpiration suction is physiologically controlled by the opening of the leaf stomata which in turn react mainly to water balance, light, and temperature. Sap flow velocity is in the order of magnitude up to approximately 1 m/h. The value refers to the flow velocity of the water or electrolyte in the conductive paths of the xylem. At night the sap flow comes to a standstill.

The sap flow is a measure for the photosynthesis performance and thus for the growth and yield of the plant. It is a plant physiological parameter that is important for agriculture and forestry. It may become particularly relevant for a "smart agriculturing" in which sensors provide information for adequate watering/irrigation.

Thermal methods have been established for determining the sap flow, above all the Granier method. It is based on the measurement of a temperature difference between two temperature sensors implanted vertically above each other in a tree trunk. The lower sensor provides a reference temperature, the upper sensor is heated by an electrical resistor and indicates an elevated temperature. Sap flow leads to a cooling of the upper sensor and thus to a reduction of the temperature difference. Via an empirical relationship, a sap flow density is calculated from the temperature difference.

There is also a known method using non-invasive sensor technology, also called "heat balance method", in which heating elements and temperature sensors are applied to a stem surface from the outside. However, this method requires a relatively complex thermal insulation and the plant when measured over a longer period of time is impaired by the missing gas exchange of the bark with the ambient air. All thermal methods described so far use at least two temperature sensors besides an additional heating element. Alternatively, the sap flow can be measured by examining the stomata opening in the photosynthesis organs. It is carried out in plant physiological research with a so called Porometer.

For a long time, plant physiological research has also known the phenomenon of the acoustic emission of plants. This branch of bioacoustics connects noises that can arise during the transport of liquid in the plants' conduit system, e.g. at cavitation, to, among other things, their water supply. However, questions of data interpretation and calibration are still unclear if the method is to be used to assess the water supply. In addition, it can be seen that even more cost-effective methods are needed which are suitable for broader field studies and long term monitoring.

Dendrometric sensors are known to measure a circumference of tree trunks with the aid of strain gauge. Dendrometric measurements, measurements of transpiration and soil water stress have shown a relationship between sap flow and stem diameter. The circumference reflects the extent of the xylem flow channels. The elastic xylem in the stem contracts as a result of the transpiration suction. Within a circadian period the changes in diameter are minimal and consequently not trivial to be reliably monitored.

It is therefore an object of the present invention to provide a method for determining a relative change in a sap flow density in trees and other vascular plants and a measurement arrangement for determining the relative change in the sap flow density such that it is ensured in a simple, reliable, and cost-effective manner that relative changes in the sap flow density can be monitored accurately over a long period of time without impairing the plant by a missing gas exchange of the bark with ambient air.

The object is solved by the method of claim 1, the computer program product of claim 10, and the measurement arrangement of claim 11. Further developments and advantageous embodiments are defined in the dependent claims.

The method according to the invention for determining a relative change in a sap flow density in a vascular plant, comprises the steps:

- arranging a first electrode and a second electrode in the vascular plant to determine an electrical impedance of xylem tissue of the vascular plant;
- either applying an alternating probe voltage over the first and second electrodes and measuring a resulting current flowing from one of the first and second electrodes to the other of the first and second electrodes or applying an alternating probe current flowing from one of the first and second electrodes to the other of the first and second electrodes and measuring a resulting voltage over the first and second electrodes;

- monitoring a thermal state in the xylem tissue while applying either the alternating probe voltage or the alternating probe current;
- calculating from either the applied alternating probe voltage and the measured resulting current or the applied alternating probe current and the measured resulting voltage a magnitude of the electrical impedance of the xylem tissue, wherein an influence of the thermal state of the xylem tissue on the magnitude of the electrical impedance is accounted for; and
- determining the relative change in the sap flow density in the vascular plant by using a relation between the sap flow density and the magnitude of the electrical impedance that an increased sap flow density results in an increased magnitude of the electrical impedance.

The first and second electrodes may be formed in the same way or different from each other. The thermal state may refer to a temperature at a location of the first electrode, the second electrode, or the space of the xylem in between both electrodes. It is also possible that the thermal state refers to an atmosphere outside of the vascular plant in the vicinity of the xylem. Although the magnitude of the electrical impedance is calculated, it may be referred to the calculation of the impedance as a measurement thereof. The relation between the increased sap flow density and the increased magnitude of the electrical impedance may be linear or non-linear depending on the vascular plant and/or the environmental conditions of the plant and/or the absolute regime of the sap flow density. The sap flow density may be in the unit $m^3/(m^2 \cdot s)$ or $g/(m^2 \cdot s)$.

The inventive method utilizes the concept of determining electrical impedance. The electrical impedance Z of the measuring object in form of the xylem tissue is calculated from the probe voltage applied to the measuring object, or an impressed probe current, and the measured resulting current, or the measured resulting voltage. Each of the terms voltage and current is used as a value or strength of the respective voltage and current. The electrical impedance contains as components the magnitude of the electrical impedance |Z| and the phase angle $\phi$ between current and voltage. In case of a bipolar impedance measurement, i.e. including two electrodes in form of the first and second electrodes on a single measuring object of xylem tissue, the measured impedance Z can be modelled as resulting in an integral over the entire volume V of the measuring object, with the product of the specific electrical resistance $\rho$ and the square of the local unit current density j' below the integral:

$$(1) \qquad Z = \iiint \rho \cdot j'^2 \, dV$$

Equation (1): $j' = j/I_1$, where j: current density $[A/m^2]$; $I_1$: total current [A]; $\rho$: specific electrical resistance $[\Omega m]$.

The sensitivity of each volume element with respect to the measured total impedance Z is in this case proportional to the local square of the normalized current density j'. This may set the framework for geometric arrangements of the electrodes: the impedance contribution of the plant tissue in the vicinity of an electrode tip with a high current density may become particularly large. For physiological measurements, the tissue around the tip should thus be as functionally intact as possible. From equation (1), it is thus preferred to derive an argument for electrodes that are as small as possible and are inserted into the plant tissue in a minimally invasive way. The term "minimally invasive" comes from the field of medicine and may be defined "as invasive as unavoidable, as less invasive as possible". In the context of the invention, the term "minimally invasive" may relate to invasions of an electrode into the vascular plant such that a result of the invasion is so small that there is no recognizable defense reaction of the plant to the invasion or such that the diameter of the electrode relative to the diameter of the stem, i.e. the diameter of the stem axis at which the measurement is made, is given e.g. by a ratio of 1/100.

For the invention, it does not matter whether the measuring arrangement is in the form of two spatially separated electrodes, for example in the form of two needle-shaped electrodes made of a non-corroding material, which may be approximately 5-10 mm long and approximately 0.25 - 2 mm in diameter, spaced apart at a distance of a few centimetres along a sprout axis of the plant, or in the form of a combination of two electrodes in a bipolar probe arranged adjacent to and isolated from each other, wherein the bipolar probe may be optionally complemented by a temperature sensor. A diameter of a plant sprout is often referred to in botany as a diameter of the "sprout axis" apart from the definition in mathematics and physics that a diameter of an axis is infinitesimally small.

If absolute quantities of the sap flow, e.g. a sap flow density in $m^3/(m^2 \cdot s)$ are to be determined, a calibration of the inventive method can yield such absolute quantities.

The calibration may comprise an assumption of a zero sap flow at night. The impedance measured at night thus corresponds to the zero point of a calibration (0%). As a second calibration point or reference value, for the same or comparable plants, the maximum impedance value occurring during the day - under optimal conditions - can be used (100%).

When using first and second electrodes of small dimensions compared to the stem diameter of the vascular plant, the measurement can be performed minimally invasive and gentle for the xylem tissue. Combinations of temperature sensors and heating coils as required with the Granier method in the state of the art are not necessary. The measuring devices are also easier to install, ideally without tools. Evaluation electronics for determining the relative change in the sap flow density in the vascular plant can be made simple, inexpensive, can be realized in a space-saving way and are therefore well suited for field use. This makes the method also suitable for area-wide long-term and remote monitoring. The current consumption of the impedance measurement can be made low, e.g. a lot smaller than 1 mA, and is required for only a short time, e.g. a few milliseconds at a typical sampling rate of e.g. 5 minutes. Thus, the device for executing the inventive method can be operated either with one or more (rechargeable) batteries, e.g. an AA battery of a typical capacity of 2000 mAh, and/or with small, inexpensive solar modules, which are energy self-sufficient and also suitable when considering the power consumption for data communication. If a higher electrical output is required, the inventive method can be carried out at lower sampling rates. The data on relative changes in the sap flow density can be combined with data on relevant, in particular climatological environmental factors, such as light and/or air temperature and/or air humidity, and can be fed into algorithms providing a better monitoring of a condition of a given vegetation of vascular plants, a prognosis of plant yields or more targeted interventions in terms of watering/irrigation. In any case, by utilizing an electrical impedance determination by measuring a voltage and current in two electrodes, it is ensured in a simple, reliable, and cost-effective manner that the relative changes in the sap flow density are monitored accurately also over a long period of time without impairing the plant by a missing gas exchange of the bark with ambient air.

In an advantageous embodiment of the inventive method, for monitoring the thermal state in the xylem tissue a temperature in the xylem tissue is measured and/or the temperature in the xylem tissue is calculated, wherein the temperature is calculated based on ambient weather conditions, such as an air temperature and/or an air humidity in the vicinity of the vascular plant, and/or ambient watering conditions, such as an amount and/or a frequency of the watering, and/or either the applied alternating probe voltage and the measured resulting current or the applied alternating probe current and the measured resulting voltage. In order to obtain precise measurements, it is thus preferred that the physical influence of the ambient temperature on the magnitude of the electrical impedance $|Z|$ is compensated. Higher temperatures at the measuring point lead to a reduction of $|Z|$ due to higher ion mobility. For exact measurements, the measuring device is therefore preferred to also provide a temperature sensor at the measuring point. Alternatively, the thermal state in the xylem tissue in form of the temperature in the xylem tissue is calculated based on ambient weather conditions without utilizing a temperature sensor added to a probe or vicinity of the first and second electrodes.

It is preferred that either a probe voltage amplitude of the applied alternating probe voltage or a probe current amplitude of the applied alternating probe current is applied such that either the measured resulting current or the measured resulting voltage remains in an essentially linear regime of a current-voltage relation of either the applied alternating probe voltage and the measured resulting current or the applied alternating probe current and the measured resulting voltage. This linear range may be a function of the sample frequency, wherein e.g. at frequencies of 100 kHz there is a linearity up to approximately 100 mA/cm$^2$. For an impedance measurement with a low excitation amplitude of the probe voltage and a size of the electrode of approximately $5 \cdot 10^{-2}$ cm$^2$, a current at 100 mV excitation at 10 kHz of approximately 1 $\mu$A at approximately $10^5$ Ohm impedance results in a current density of 0.02 mA/cm$^2$, i.e. also at 10 kHz it is measured still in the linear regime of a current-voltage relation.

Particularly in this linear range, the excitation amplitude of the resulting current or the probe current can be a lot smaller than 1 mA, e.g. 0.1 - 0.01 mA, leading to a low current consumption of the impedance measurement which may require only a short period of time, e.g. a few milliseconds at a typical sampling rate of e.g. 5 minutes as stated above. Operation by using a (rechargeable) battery, such as an AA or AAA battery, and/or small, inexpensive solar modules, which are energy self-sufficient and are able to also cover the power consumption for data communication becomes possible. When the electrical impedance of the xylem tissue of the vascular plant is determined over time, the relative change in the sap flow density in the vascular plant can be determined advantageously by using a proportionality that a circadian amplitude of daily fluctuations of the magnitude of the electrical impedance is proportional to the sap flow density. This proportionality may be linear or non-linear depending on the vascular plant and/or the environmental conditions of the plant and/or the absolute regime of the sap flow density. The relative change in the sap flow density in the vascular plant over at least two days may be determined by analysing the superimposed circadian variation of the electrical impedance $\Delta |Z|_d$ after calibration with an empirical relationship, e.g. a condition of the stomata apparatus. This way, by just determining the electrical impedance over time both a hydraulic status of the plant, e.g. a general water supply which is measurable by a daily mean value of the impedance magnitude $|Z|$ and the sap flow determined directly from the amplitude of the circadian variation $\Delta|Z|_d$ can be monitored.

By determining the electrical impedance of the xylem tissue of the vascular plant over at least two days, a quantitative indicator for a drought stress of the vascular plant can be generated such that an increase in the drought stress results in an average increase of the magnitude of the electrical impedance over the at least two days, and the relative change in the sap flow density in the vascular plant is determined taking into account additional information about a species-specific reaction of the stomata apparatus of the vascular plant on the drought stress, i.e. a relation between the drought stress and the sap flow density for the vascular plant. The electrical impedance as the primary measured signal may be translated into the required physiological parameter of drought stress by an empirical relationship. An example for an independent indicator of drought stress useable for calibration is the condition of the stomata apparatus. Besides the measurement of the hydraulic status of the plant, e.g. the general water supply, measurable by the daily mean value of the impedance magnitude |Z|, the sap flow can be derived indirectly from data on the relationship between the drought stress and the transpiration. For plausibility measures, the derived sap flow can be compared with the sap flow determined directly from the amplitude of the circadian variation $\Delta|Z|_d$.

It is preferred for exact more accurate measurements that an influence of the thermal state of the xylem tissue on the magnitude of the electrical impedance is accounted for in that the magnitude of the electrical impedance is corrected for a dependence of the magnitude of the electrical impedance on a temperature of the xylem tissue.

In a further advantageous embodiment of the invention either a probe voltage amplitude of the applied alternating probe voltage or a probe current amplitude of the applied alternating probe current is applied such that either the measured resulting current or the measured resulting voltage is in an essentially non-linear regime of a current-voltage relation of either the applied alternating probe voltage and the measured resulting current or the applied alternating probe current and the measured resulting voltage. An impact of the thermal state of the xylem tissue on the magnitude of the electrical impedance may be accounted for in that an increase of a temperature in the xylem tissue, e.g. by approx. 5 - 10 °C depending on a current density in the xylem tissue excited by either the measured resulting current or the applied alternating probe current, is caused by an electrical power which is released into the xylem tissue and predetermined by either the probe voltage amplitude of the applied alternating probe voltage or the probe current amplitude of the applied alternating probe current. Alternatively, the increase of a temperature in the xylem tissue may be predetermined and caused by either the probe voltage amplitude of the applied alternating probe voltage or the probe current amplitude of the applied alternating probe current as required for the predetermined increase in temperature.

In the case of excitation with a fixed current amplitude $I_2$ of the probe current, the following relationship applies to the active power (electrical instead of active?) P converted in the measuring volume V:

$$(2) \qquad P = \iiint \rho \cdot j^2 \cdot \cos \Phi \, dV$$

Equation (2) j: current density [A/m2]; p: specific electrical resistance [$\Omega$m]; $\Phi$: phase angle between current and voltage.

The power P is thus generated proportional to the value of the specific electrical resistance and proportional to the square of the local current density. The resulting local temperature increase depends on the thermal material properties of the surrounding plant tissue, such as thermal conductivity and specific heat capacity, and increases nonlinearly with the current density. At a given constant electrical power, it takes a certain amount of time for the system to reach thermal equilibrium and a constant (over-) temperature profile to set up. An ionic conductivity of plant tissue is temperature-dependent, wherein the ion equivalent conductivity of $Na^+$ and $Cl^-$ increases by about 50% between 20°C and 40°C. The impedance is reciprocal to the conductivity. The sap flow of the surrounding plant tissue has the effect of cooling the locally heated tissue and thus increasing its electrical impedance compared to a situation without sap flow. In other words: The dissipation of heat into the environment is increased by sap flow. An advantage for the sensitivity of the measurement is that the sites of the greatest temperature increase, e.g. the electrode tips, are also the sites that make the greatest contribution to the measured electrical impedance of the plant tissue. If a large excitation amplitude is used, the over-temperature effect overlaps with a contraction effect of the vascular bundles. The sum of both of these effects may lead to an increase of the overall sensitivity to the detection of the relative changes in the sap flow density compared to a small excitation amplitude which can affect only the contraction effect of the vascular bundles without causing in addition an over-temperature effect.

Determining relative changes in the sap flow density in the non-linear regime of the current-voltage relation may require a higher electrical output of the power supply compared to determinations of the sap flow in the linear regime of the current-voltage relation so that the former measurements are more likely to be carried out at lower sampling rates than the latter measurements.

Advantageously, a constant over-temperature profile may be set up at a predetermined constant electrical power in the xylem tissue after a thermal equilibrium is reached, such that the sap flow density of a surrounding xylem tissue effects a cooling of the xylem tissue showing the over-temperature profile, e.g. in the form of a locally heated xylem tissue, and results in an increase of the magnitude of the electrical impedance compared to a magnitude of the electrical impedance determined without a sap flow. At a given constant electrical power, it takes a certain amount of time for the system to reach thermal equilibrium and a constant over-temperature profile to set up. In other words: The sap flow is assumed to be variable over time, which is monitored by the measured impedance. If the thermal equilibrium has been reached at a certain sap flow, i.e. sap flow density, a certain impedance is measured between the two electrodes. If the sap flow increases, the measuring point is cooled and the impedance should increase. This way, relative changes in the sap flow density can be accurately and reliably determined.

When either the magnitude of the electrical impedance varying depending on the sap flow density is determined or either the probe voltage amplitude of the applied alternating probe voltage or the probe current amplitude of the applied alternating probe current is controlled, e.g. via a measuring bridge, such that the magnitude of the electrical impedance remains essentially constant, an accuracy and reliability of the determination of the relative changes of the sap flow density can be further improved. In the second alternative, an increasing impedance is compensated by a reduction of the excitation amplitude until a predetermined impedance value is reached. To obtain a primary measurement signal, either the magnitude of the electrical impedance varying depending on the sap flow density is determined at a constant amplitude of the excitation source or the probe voltage amplitude or the probe current amplitude of the applied excitation source is controlled, e.g. via a measuring bridge, such that the magnitude of the electrical impedance remains essentially constant. In the latter solution, an increase of impedance due to a cooling effect by sap flow is compensated by an increased amplitude of the excitation the excitation source until a predetermined level of impedance is reached.

Another aspect of the invention is a software program realizing the methods according to the invention when executed on a computer. In the aforementioned software program the computer is preferably a distributed computing system wherein part of the computing system is located / arranged / operated in a cloud computing system. The software program may be embodied as a computer program product or a data carrier carrying data representing the software program.

The invention also relates to a measurement arrangement for determining a relative change in a sap flow density in a vascular plant. The measurement arrangement comprises:

- an electrical impedance sensor configured such that a first electrode and a second electrode are arranged in the vascular plant to determine an electrical impedance of xylem tissue of the vascular plant;
- a power supply and electrical measurement means configured to either apply an alternating probe voltage over the first and second electrodes and measure a resulting current flowing from one of the first and second electrodes to the other of the first and second electrodes or to apply an alternating probe current flowing from one of the first and second electrodes to the other of the first and second electrodes and measure a resulting voltage over the first and second electrodes;
- thermal monitoring means configured to monitor a thermal state in the xylem tissue while applying either the alternating probe voltage or the alternating probe current;
- calculating means configured to calculate from either the applied alternating probe voltage and the measured resulting current or the applied alternating probe current and the measured resulting voltage a magnitude of the electrical impedance of the xylem tissue, wherein an influence of the thermal state of the xylem tissue on the magnitude of the electrical impedance is accounted for; and
- determination means configured to determine the relative change in the sap flow density in the vascular plant by using a relation between the sap flow density and the magnitude of the electrical impedance that an increased sap flow density results in an increased magnitude of the electrical impedance.

The measurement arrangement can be integrated in one and the same simple measuring device. Power supply can be provided in form of a e.g. an AA battery which may provide power/electrical energy also for communicating the acquired relative changes in the sap flow density and/or absolute values of the sap flow density and possibly environmental data to a receiving unit.

It is preferred that the electrical impedance sensor is configured such that the first electrode and the second electrode are either spatially separated from each other or arranged adjacent to and partially isolated from each other. This way, the impedance sensor is configured in a simple and reliable manner.

With advantage, the first and second electrodes may be two spatially separated needle-shaped electrodes, e.g. made of a non-corroding material, approximately 5-10 mm long, approximately 0.25 - 2 mm in diameter, respectively, and spatially separated from each other by approximately 1 - 10 centimeters along a sprout axis of the vascular plant. A reliable and cost-effective sensor which is e.g. scalable for a multitude of vascular plants being probed e.g. at the same time, is provided.

It is particularly preferred that the electrical impedance sensor is a bipolar measurement probe, wherein the first electrode of an elongated form is sandwiched between the second electrode and a single temperature sensor, wherein a first tip portion of the first electrode projects compared to a second tip portion of the second electrode and a third tip portion of the temperature sensor in a direction of insertion into the xylem tissue, wherein an interface between any pair of the first electrode, the second electrode, and the temperature sensor is isolated from each other such that the projecting first tip portion is bare to be inserted into the xylem tissue along with the bare second tip portion of the second electrode and the bare third tip portion of the temperature sensor. A compact and accurate impedance sensor allowing a compensation of the influence of temperature on the ion conductivity by just one temperature sensor in close proximity to the first electrode, is thus provided.

If at least one of the first and second electrodes has a tip showing dimensions that are small compared to a diameter of a stem of the vascular plant, e.g. such that a ratio of a diameter of the tip to the diameter of a stem of the vascular plant is approximately 1/100, a minimally invasive insertion of the tip into the vascular plant becomes possible. Moreover, the impedance contribution of the plant tissue in the vicinity of such an electrode tip with a potential of providing a high current density becomes particularly large leading to an improved accuracy in the impedance measurement due to a larger signal-to-noise ratio compared to a broader dimensioned electrode tip allowing only smaller current densities [see above discussion of equation (1)].

Other aspects, features and advantages of the invention will become apparent by the below description of exemplary embodiments alone or in cooperation with the appended drawings.

Fig. 1    is a side view of first and second electrodes inserted into a trunk of a young pine according to a first embodiment of the invention,

Fig. 2    is a schematic view of a bipolar measuring probe according to a second embodiment of the invention, wherein in addition to the first and second electrodes a temperature sensor is arranged adjacent to the first electrode to measure an ambient temperature,

Fig. 3    is a schematic diagram including functional units of a measurement arrangement according to another embodiment of the invention, wherein the temperature sensor 9 connected to the thermal monitoring means 17 is optional,

Fig. 4    is a schematic representation of the influence of environmental factors and the plant physiology on the magnitude of the electrical impedance determined by the method for determining a relative change in a sap flow density in a vascular plant according to the invention,

Fig. 5    is a graphic representation of a daily variation of the magnitude of the electrical impedance over a time period of two days, wherein a circadian amplitude of daily fluctuations of the magnitude of the electrical impedance is proportional to the sap flow density according to a further embodiment of the invention, and

Fig. 6    is another graphic representation of a daily variation of the magnitude of the electrical impedance over a longer time period than that shown in Fig. 5, i.e. over two months, wherein a soil moisture is measured concurrently with the magnitude of the electrical impedance as part of a nine-week measurement on a young pine in a plant pot.

Now, exemplary embodiments of the invention will be described in further detail.

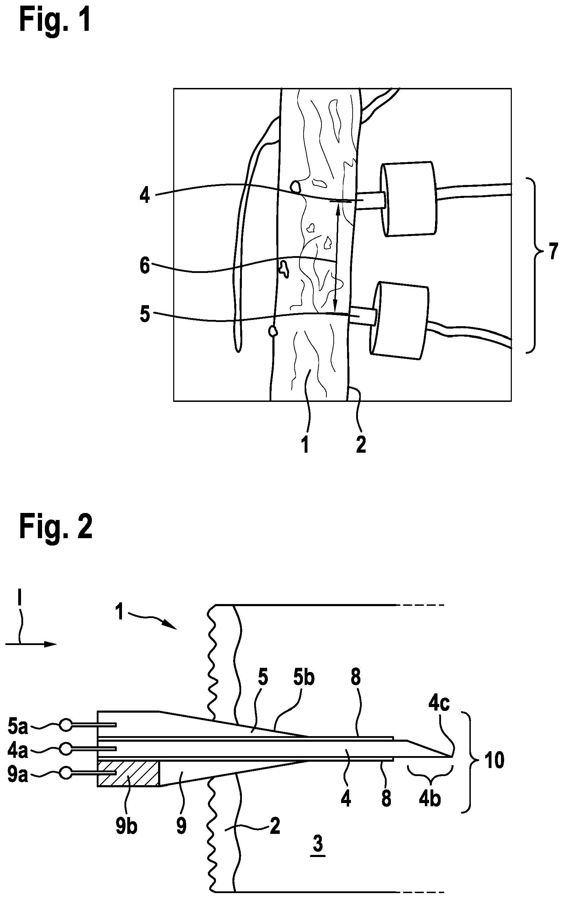

Fig. 1 shows a side view of first and second electrodes 4, 5 inserted into a trunk of a young pine as an example of a vascular plant 1 according to an embodiment of the invention. Tips of the first and second electrodes 4, 5 (not shown as being inserted into the trunk of the plant 1) are spatially separated by a spatial distance 6 of a few centimeters along a sprout axis of the vascular plant 1. Both electrodes 4, 5 are part of an impedance sensor 7, wherein the material of each of the electrodes 4, 5 is noncorrosive, e.g. made from stainless steel. The tips of both electrodes 4, 5, when being inserted into the vascular plant 1, initially pass a phloem tissue 2 in the bark area at the outside of the vascular plant 1 before entering a xylem tissue in the inner region of the vascular plant 1. The tips of the electrodes 4, 5 may be needle-shaped, approximately 5 to 10 mm long, and approximately 0.25 to 2 mm in diameter.

Fig. 2 shows a schematic view of a bipolar measuring probe 10 in accordance to a second embodiment of the invention, wherein the probe 10 is inserted into the vascular plant 1 in an insertion direction I. In addition to the first and second electrodes 4, 5, a temperature sensor 9 is arranged adjacent to the first electrode 4 to measure an ambient temperature by using the temperature sensor element 9b. The first electrode 4 comprises a tip region 4b and a tip 4c, wherein the tip region 4b and the tip 4c are inserted in the xylem tissue 3 of the vascular plant 1 after having passed through the phloem tissue 2 of the vascular plant 1. The bipolar measuring probe 10 comprises an inner electrode as the first electrode 4 which is longitudinally extended in the insertion direction I, wherein on a side opposing the side of the first electrode 4 including the tip region 4b and the tip 4c there is connected a contact interface 4a in form of an open wire to the first electrode 4. The second electrode 5, which is arranged above the first electrode 4, comprises a contact interface 5a in form of another open wire, wherein the second electrode 5 is formed as a wedge which is partially inserted into the vascular plant 1 such that a tip region 5b is located in the xylem tissue of the vascular plant 1. The temperature sensor 9 may also be formed as a wedge as indicated in Fig. 2 which is partially inserted into the xylem tissue 3 of the vascular plant 1 although the temperature sensor element 9b of the temperature sensor 9 is arranged outside of the vascular plant 1 to provide data about an ambient air temperature of the plant 1.

The first tip portion 4b of the first electrode 4 projects compared to the second tip portion 5b of the second electrode 5 and a third tip portion of the temperature sensor 9 in the direction of insertion I into the xylem tissue 3. An interface between the first electrode 4 and the second electrode 5 as well as another interface between the first electrode 4 and the temperature sensor 9 are isolated from each other by using an isolation 8 in form of an isolating layer. Just adjacent interfaces between the first electrode 4, the second electrode 5, and the temperature sensor 9 are isolated from each other such that the projecting first tip portion 4b in the direction of insertion I is bare to be inserted into the xylem tissue along with the bare second tip portion 5b of the second electrode 5 and the bare third tip portion of the temperature sensor 9. Although the temperature sensor element 9b provides data about the ambient air temperature outside of the vascular plant 1, it is possible to measure the inner temperature of the vascular plant 1 in the xylem tissue 3 in close proximity to the tip 4c of the first electrode 4. Therefore, the temperature sensor 9 comprising a contact interface 9a may be used for measuring the internal temperature of the vascular plant 1 in the xylem tissue 3 and/or the outside temperature of the vascular plant 1 as the ambient air temperature.

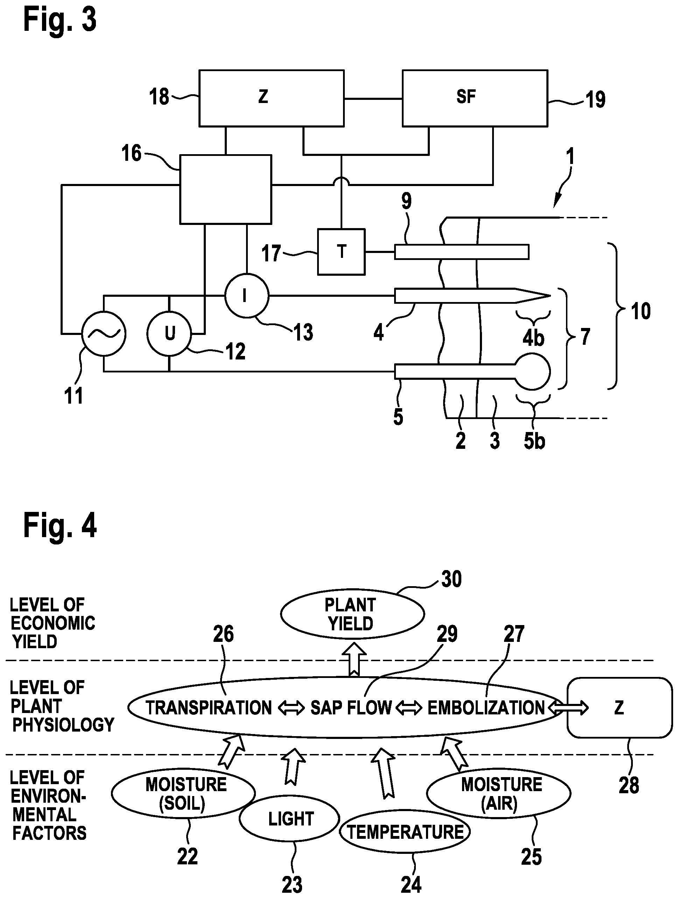

Fig. 3 is a schematic diagram including functional units of a measurement arrangement according to a further embodiment of the invention. Two spatially separated first and second electrodes 4, 5 are inserted into the vascular plant 1 by passing both electrodes partially through the phloem tissue 2 into the xylem tissue 3 so that the first tip region 4b of the first electrode 4 and the second tip region 5b of the second electrode 5 are arranged in the xylem tissue 3. While other shapes of the tip regions 4b and 5b are possible, the tip region 4b of the first electrode 4 is needle-shaped, whereas the form of the second tip region 5b of the second electrode 5 is sphere-shaped. This way, a current density is larger at the tip of the tip region 4b of the first electrode 4 compared to a current density generated at the second tip region 5b of the second electrode 5. Consequently, due to the larger current density at the first tip region 4b of the first electrode 4, the first electrode 4 contributes to a larger extend to an impedance Z and as a result a magnitude of the electrical impedance |Z|. The dependence of the electrical impedance Z on current density is such that the electrical impedance Z is proportional to the square of the current density leading to a major contribution of the electrical impedance Z provided by the current density at the first tip region 4b of the first electrode 4 compared to the current density provided by the second 5b of the second electrode 5 (see also discussion of equation (1) above).

The electrical impedance sensor 7 including only the first and second electrodes 4, 5 (see Fig. 1) may be supplemented by the temperature sensor 9 which is partially inserted into the vascular plant 1 with its tip being inserted in the xylem tissue 3, wherein the temperature sensor 9 is arranged in close proximity to the first electrode 4 in order to be able to measure the temperature in the xylem tissue 3 affected by the tip region 4b and/or outside of the vascular plant 1 in close proximity to the first electrode 4. When the first and second electrodes 4, 5 are combined with the temperature sensor 9, another electrical impedance sensor 10 may be formed (see also Fig. 2). A power supply and electrical measurement means 16 are configured to either apply an alternating probe voltage 12 over the first and second electrodes 4, 5 and measure a resulting current 13 flowing from one of the first and second electrodes 4, 5 to the other or to apply an alternating probe current from one of the first and second electrodes 4, 5 to the other and measure a resulting voltage over the first and second electrodes 4, 5. The first and second electrodes 4, 5 are connected by a power supply 11 which in the embodiment shown in Fig. 3 provides an alternating probe voltage 12 over the first and second electrodes 4, 5.

Thermal monitoring means 17 are connected to the temperature sensor 9 and configured to monitor a thermal state in the xylem tissue 3 in form of a temperature in the xylem tissue 3 while the alternating probe voltage 12 is applied to the xylem tissue 3 to determine an electrical impedance Z of the xylem tissue 3 of the vascular plant 1. From the applied alternating probe voltage 12 and the measured resulting current 13 a magnitude of the electrical impedance |Z| is calculated by the calculating means 18, wherein an influence of the thermal state of the xylem tissue 3 on the magnitude of the electrical impedance |Z| in form of the temperature in the xylem tissue 3 is accounted for. More specifically, an ion mobility which is dependent on temperature such that a larger temperature leads to a larger ion mobility which in turn leads to a smaller magnitude of impedance |Z| is corrected for the temperature in the xylem tissue 3 measured by the temperature sensor 9 and transmitted to the thermal monitoring means 17 for being taken into account at the calculating means 18. Determination means 19 are connected to the calculating means 18, the thermal monitoring means 17, and the power supply electrical measurement means 16 to determine a relative change in the sap flow density in the vascular plant 1 by using a relation between the sap flow density and the magnitude of the electrical impedance |Z| that an increase in sap flow density results in an increased magnitude of the electrical impedance |Z|. While in the embodiment shown in Fig. 3 the temperature sensor 9 is connected to thermal monitoring means 17 being connected to the calculating means 18 and the determination means 19, the temperature sensor 9 is not an essential feature of the invention. To the contrary, it is possible, instead of using the temperature sensor 9, e.g. in the vicinity of the probed xylem tissue 3, to calculate or approximate the temperature in the xylem tissue, wherein such a temperature is based on ambient weather conditions, e.g. an air temperature and/or an air moisture in the vicinity of the vascular plant 1, and/or ambient watering conditions, such as an amount and/or a frequency of the watering, and/or either the applied alternating probe voltage 12 and the measure resulting current 13 or the applied alternating probe current and the measure resulting voltage.

In accordance to the embodiment shown in Fig. 3, a magnitude of the electrical impedance |Z| is thus measured. Over both electrodes 4, 5 an alternating voltage 12 with a fixed parameter setting may be applied. This can be done with a defined, relatively low amplitude of the excitation such that either the measured resulting current 13 or the measured resulting voltage remains in an essentially linear regime of a current-voltage relation of either the applied alternating probe voltage 12 and the measured resulting current 13 or the applied alternating probe current and the measured resulting voltage. The essentially linear regime of the current-voltage relation may be reached at a defined frequency of excitation, e. g. between 1 and 100 kHz. The magnitude of the electrical impedance |Z| is inversely proportional to the specific electrical conductivity of the material in form of the xylem tissue 3 arranged in between both electrodes 4, 5. Dry wood has a relatively low conductivity, i.e. a large magnitude of electrical impedance |Z|, and an electrolyte exhibits a relatively high conductivity, i.e. a small magnitude of electrical impedance |Z|.

Water transport in the xylem tissue 3 is driven by a difference in water potential between soil and atmosphere. In case of an open stomata in the plant's transpiration organs, whose opening is controlled by light and a number of other external factors (see Fig. 4 below), this difference ensures a suction effect and sap flow from the root area to the leaves. The strength of the negative pressure also causes a contraction of the vascular bundles of the vascular plant 1. The diameter of the stem axis and the mass of the water per volume element of the plant tissue, i.e. the water density, are directly related to the contraction of the vascular bundles. The electrical impedance Z is inversely proportional to the water density in the measurement volume. Contractions/dilatations of the vascular bundles can thus be measured via the magnitude of the electrical impedance | Z |.

The plant physiological correlations are described in Fig. 4. This figure is a schematic representation of the influence of environmental factors and the plant physiology on the magnitude of the electrical impedance |Z| determined by the method for determining the relative change in the sap flow density in a vascular plant 1 according to the invention. A light-induced opening of the leaf stomata sets a circadian period of transpiration in motion. Day light 23 creates a suction effect in the stem that leads to contraction of the vascular bundles, i.e. embolization 27, which in turn leads to an increase in the electrical impedance Z, 28. In addition to light 23, transpiration 26 is also controlled by temperature 24, humidity in the form of air moisture 25, and soil moisture 22. Therefore, transpiration 26 creates a suction effect in the stem, leads to the contraction of the vascular bundles in form of embolization 27, leading to an increase in the impedance 28. The sap flow 29 and with it the sap flow density depends on the level of contraction of the vascular bundles in form of the embolization 27 which in turn is measured by the electrical impedance Z, 28. Depending on the plant species, the stomata closes differently sensitive in reaction to drought stress and thus restricts transpiration 26. However, this restriction of transpiration 26 also limits the gas exchange rate, the net photosynthesis rate and thus the plant yield 30 of the vascular plant 1 decreases.

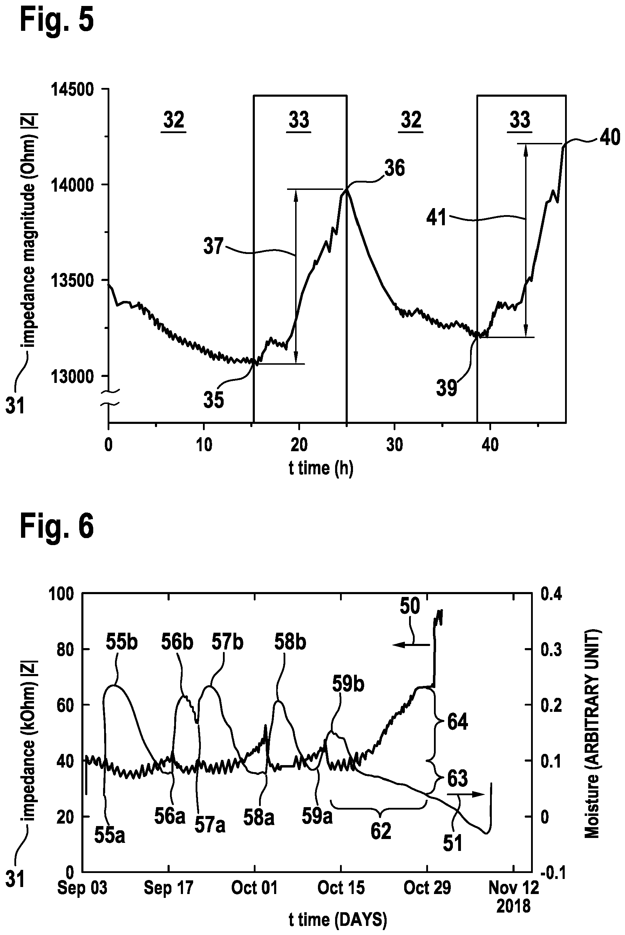

Fig. 5 shows an example of daily fluctuations of the magnitude of the electrical impedance |Z|. The figure is a graphic representation of a daily variation of the magnitude of the electrical impedance 31 over a period of two days. A circadian amplitude $\Delta|Z|_d$ of daily fluctuations of the magnitude of the electrical impedance |Z|, 31, is proportional to the sap flow density according to a further embodiment of the invention.

The circadian amplitude of daily fluctuations $\Delta|Z|_d$ of the magnitude of the electrical impedance 31 being proportional to the sap flow density allows to determine relative changes of the sap flow density and subsequent to a calibration a determination of absolute values of the sap flow density. The time scale in hours shows regions of night time 32 and regions of day time 33. The determined magnitude of the electrical impedance |Z|, 31, exhibits daily variations including minima, i.e. minimum amplitudes, 35, 39 at the end of the night time regions 32 and maxima, i.e. maximum amplitudes, 36, 40 at the end of the day time regions 33. The circadian cycle variation can be determined by deducing from the amplitude of the electrical impedance maximum 36 the amplitude of the electrical impedance minimum 35 as the first value 37. The day after, the differential amplitude of the electrical impedance between the maximum 40 and the minimum 39 can be deduced as a second circadian amplitude 41 of daily fluctuations of the magnitude of the electrical impedance 31. Both circadian amplitudes 37, 41 are approximately 1 k$\Omega$ which accounts for about 7.5 % of the absolute magnitude of the electrical impedance 31. The circadian amplitudes 37, 41 have been found to be proportional to the sap flow density allowing a determination of relative changes and absolute values of the sap flow density by just determining the circadian amplitude $\Delta|Z|_d$ of the magnitude of the electrical impedance |Z|, 31.

Along with the circadian fluctuations of the sap flow also the general water supply of the vascular plant 1 has an influence on the magnitude of the electrical impedance |Z|, which may be due to an incipient cavitation and embolization in the xylem tissue 3 at water scarcity, accompanied by a reduction of the water density in the measuring volume. Due to the influence of the general water supply on the vascular plant 1 in addition to the circadian fluctuations of the sap flow density the absolute values of the magnitude of the electrical impedance |Z| are not constant over time.

To this end, Fig. 6 is another graphic representation of a daily variation of the magnitude of the electrical impedance |Z| over a longer period of nine weeks. A soil moisture in arbitrary units, indicated by arrow 51, is measured concurrently with the magnitude of the electrical impedance 31, as indicated by arrow 50, wherein the measurements of soil moisture and impedance 31 have been part of a nine-week measurement on a young pine in a plant pot. Increasing water scarcity manifests itself in rising values of the magnitude of the electrical impedance |Z| on the longer, multi-day time scale shown in Fig. 6. The young pine as an example of a vascular plant 1 was watered on September 5, 2018, 55a, September 17, 2018, 56a, September 21, 2018, 57a, October 2, 2018, 58a, and on October 12, 2018, 59a. Following these watering events, the soil moisture steeply increased showing maxima 55b, 56b, 57b, 58b, and 59b about one day after the respective water event. In addition to the circadian fluctuations $\Delta|Z|_d$ with the daily maxima and minima of the magnitude of the electrical impedance |Z|, a trend revealing an increase 64 of the magnitude of the electrical impedance |Z|, 31, between the waterings/irrigations due to the drying out of the substrate and the associated water shortage of the tree with increasing water shortage as indicated by a decreasing moisture 63 can be observed. At the same time, the circadian amplitude $\Delta|Z|_d$ decreases with increasing drought as can be seen in the period 62 between October 13, 2018 and October 28, 2018.

The properties shown in Fig. 6 suggest that from two parameters of a technically simple impedance measurement conclusions can be drawn about the physiology of the vascular plant 1:

- a measurement of the circadian amplitude $\Delta|Z|_d$ provides a direct access to the sap flow, e. g. in the form of the sap flow density, of the vascular plant 1,

- by measuring the magnitude of the electrical impedance |Z|, 31, over a period of at least two days, a quantitative indicator for the drought stress of the vascular plant 1 is provided. When additional information about the species specific reaction of the stomata apparatus is retrieved, e.g. from databases or by calibration measurements, a relation between the magnitude of the electrical impedance |Z|, 31, and the sap flow density can be established from the measured magnitude of the electrical impedance.

Since the circadian fluctuations $\Delta|Z|_d$, 37, 41, may be relatively small, e.g. approximately a few percent of the absolute impedance of about 1 to 5 k$\Omega$, a low-noise impedance measurement is advantageous in order to reliably measure these circadian fluctuations. The sampling rate should be sufficient to follow the dynamics of the daily sap flow. In addition the measuring range of the absolute impedance should remain within a relatively narrow tolerance limit. Also, time scales from weeks to months should exhibit only a negligible drift in the absolute values of the magnitude of the electrical impedance 31. This in turn may be accomplished by the minimally invasive implantation/invasion of needle-shaped tips 4c of the first and/or second electrodes 4, 5, which is advantageous since the minimally invasive implantation/invasion reduces the vascular plant's defense reactions to a minimum.

The impedance sensors 7, 10 shown in figures 1, 2, the measurement arrangement shown in Fig. 3, the circadian fluctuations $\Delta|Z|_d$ shown over two days in Fig. 5, and the data on magnitudes of the electrical impedance 31 measured over a period of several days as a quantitative indicator for drought stress of the vascular plant 1 shown in Fig. 6 may be measured in the essentially linear regime of the current-voltage relation of either the applied alternating probe voltage 12 and the measure resulting current 13 or the applied alternating probe current and the measure of resulting voltage. Alternatively, the impedance sensors 7, 10 of figures 1, 2, the measurement arrangement of Fig. 3, the circadian fluctuations $\Delta|Z|_d$, 37, 41 shown in Fig. 5 and the measured magnitude of the electrical impedance 31 over a period of several days as a quantitative indicator for drought stress shown in Fig. 6 may be measured in the essentially non-linear regime of the current-voltage relation of either the applied alternating probe voltage 12 and the measure resulting current 13 or the applied alternating probe current and the measured resulting voltage. In particular, instead of using a relatively small probe voltage amplitude or a probe current amplitude in order to stay in the essentially linear regime of the current-voltage relation, it is possible to use a pre-determined constant electrical power in the xylem tissue 3 to set up a constant over-temperature profile after a thermal equilibrium between the over-temperature profile and the sap flow is reached, wherein the sap flow density of the surrounding xylem tissue 3 effects a cooling of the xylem tissue 3 showing the over-temperature profile resulting in an increase of the magnitude of the electrical impedance 31 compared to a magnitude of the electrical impedance 31 determined without a sap flow.

A technical feature or several technical features which has/have been disclosed with respect to a singular or several embodiments disclosed herein before, e.g. the insulation 8 between adjacent first and second electrodes 4, 5 or between first electrode 4 and the temperature sensor 9, may be present also in another embodiment, e.g. the impedance sensor 7 shown in figure 1 where the first and second electrodes 4, 5 are specially separated by a distance 6 of a few centimeters, except it is/they are specified not to be present or it is impossible for it/them to be present for technical reasons.

LIST OF REFERENCE SIGNS

| 1 | vascular plant |
|---|---|
| 2 | phloem tissue |
| 3 | xylem tissue |
| 4 | first electrode |
| 4a | contact interface |
| 4b | tip region |
| 4c | tip |
| 5 | second electrode |
| 5a | contact interface |
| 5b | second tip region |
| 6 | spatial distance |
| 7, 10 | impedance sensor |
| 8 | insulation |
| 9 | temperature sensor |
| 9a | contact interface |
| 9b | temperature sensor element |
| 11 | power supply |
| 12 | probe voltage |
| 13 | resulting current |
| 16 | electrical measurement means |
| 17 | thermal monitoring means |
| 18 | calculating means |
| 19 | determination means |
| 22 | soil moisture |
| 23 | day light |
| 24 | temperature |
| 25 | air moisture |
| 26 | transpiration |
| 27 | embolization |
| 28 | impedance |
| 29 | sap flow |
| 30 | plant yield |
| 31 | electrical impedance |
| 32 | night time region |
| 33 | day time region |
| 35, 39 | electrical impedance minimum amplitude |
| 36, 40 | electrical impedance maximum amplitude |
| 37,41 | circadian amplitude |
| 50 | arrow |
| 51 | arrow |
| 55a - 59a | watering event |
| 55b - 59b | soil moisture maxima |
| 62 | time period |
| 63 | period of decreasing moisture |
| 64 | period of increasing electrical impedance |
| I | direction of insertion |

**Claims**

1. Method for determining a relative change in a sap flow density in a vascular plant (1), wherein the method comprises the steps:

   - arranging a first electrode (4) and a second electrode (5) in the vascular plant (1) to determine an electrical impedance of xylem tissue (3) of the vascular plant (1);
   - either applying an alternating probe voltage (12) over the first and second electrodes (4, 5) and measuring a resulting current (13) flowing from one of the first and second electrodes (4, 5) to the other of the first and second electrodes (4, 5) or applying an alternating probe current flowing from one of the first and second electrodes

(4, 5) to the other of the first and second electrodes (4, 5) and measuring a resulting voltage over the first and second electrodes (4, 5);
- monitoring a thermal state in the xylem tissue (3) while applying either the alternating probe voltage (12) or the alternating probe current;
- calculating from either the applied alternating probe voltage (12) and the measured resulting current (13) or the applied alternating probe current and the measured resulting voltage a magnitude of the electrical impedance (31) of the xylem tissue (3), wherein an influence of the thermal state of the xylem tissue (3) on the magnitude of the electrical impedance (31) is accounted for; and
- determining the relative change in the sap flow density in the vascular plant (3) by using a relation between the sap flow density and the magnitude of the electrical impedance (31) that an increased sap flow density results in an increased magnitude of the electrical impedance (31).

2. Method of claim 1, wherein for monitoring the thermal state in the xylem tissue (3) a temperature in the xylem tissue (3) is measured and/or calculated based on ambient weather conditions.

3. Method of claim 1 or claim 2, wherein either a probe voltage amplitude of the applied alternating probe voltage (12) or a probe current amplitude of the applied alternating probe current is applied such that either the measured resulting current (13) or the measured resulting voltage remains in an essentially linear regime of a current-voltage relation of either the applied alternating probe voltage (12) and the measured resulting current (13) or the applied alternating probe current and the measured resulting voltage.

4. Method of claim 3, wherein the electrical impedance of the xylem tissue (3) of the vascular plant (1) is determined over time, wherein the relative change in the sap flow density in the vascular plant (1) is determined by using a proportionality that a circadian amplitude (37, 41) of daily fluctuations of the magnitude of the electrical impedance (31) is proportional to the sap flow density.

5. Method of claim 3 or claim 4, wherein by determining the electrical impedance of the xylem tissue (3) of the vascular plant (1) over at least two days a quantitative indicator for a drought stress of the vascular plant (1) is generated such that an increase in the drought stress results in an average increase of the magnitude of the electrical impedance (31) over the at least two days, and the relative change in the sap flow density in the vascular plant (1) is determined taking into account additional information about a species-specific reaction of the stomata apparatus of the vascular plant (1) on the drought stress.

6. Method of any of claims 1 to 5, wherein an influence of the thermal state of the xylem tissue (3) on the magnitude of the electrical impedance (31) is accounted for in that the magnitude of the electrical impedance (31) is corrected for a dependence of the magnitude of the electrical impedance (31) on a temperature of the xylem tissue (3).

7. Method of claim 1 or claim 2, wherein either a probe voltage amplitude of the applied alternating probe voltage (12) or a probe current amplitude of the applied alternating probe current is applied such that either the measured resulting current (13) or the measured resulting voltage is in an essentially non-linear regime of a current-voltage relation of either the applied alternating probe voltage (12) and the measured resulting current (13) or the applied alternating probe current and the measured resulting voltage, wherein an electrical power is released into the xylem tissue by either a predetermined probe voltage amplitude of the applied alternating probe voltage (12) or a predetermined probe current amplitude of the applied alternating probe current causing an increase of a temperature of preferably 5-10°C.

8. Method of claim 7, wherein at a predetermined constant electrical power in the xylem tissue (3) a constant over-temperature profile is set up after a thermal equilibrium is reached, wherein the sap flow density of a surrounding xylem tissue (3) effects a cooling of the xylem tissue (3) showing the over-temperature profile and results in an increase of the magnitude of the electrical impedance (31) compared to a magnitude of the electrical impedance (31) determined without a sap flow.

9. Method of claim 7 or claim 8, wherein either the magnitude of the electrical impedance (31) is determined or either the probe voltage amplitude of the applied alternating probe voltage (12) or the probe current amplitude of the applied alternating probe current is controlled, such that the magnitude of the electrical impedance (31) remains essentially constant.

10. A software program realizing the method according to any of the preceding claims when executed on a system comprising a measurement arrangement according to claim 11 and a computer, wherein the computer is preferably a distributed computing system, part of which being located in a cloud computing system.

11. Measurement arrangement for determining a relative change in a sap flow density in a vascular plant (1), wherein the measurement arrangement comprises:

- an electrical impedance sensor (7, 10) configured such that a first electrode (4) and a second electrode (5) are arranged in the vascular plant (1) to determine an electrical impedance of xylem tissue (3) of the vascular plant (1);
- a power supply and electrical measurement means (16) configured to either apply an alternating probe voltage (12) over the first and second electrodes (4, 5) and measure a resulting current (13) flowing from one of the first and second electrodes (4, 5) to the other of the first and second electrodes (4, 5) or to apply an alternating probe current flowing from one of the first and second electrodes (4, 5) to the other of the first and second electrodes (4, 5) and measure a resulting voltage over the first and second electrodes (4, 5);
- thermal monitoring means (17) configured to monitor a thermal state in the xylem tissue (3) while applying either the alternating probe voltage (12) or the alternating probe current;
- calculating means (18) configured to calculate from either the applied alternating probe voltage (12) and the measured resulting current (13) or the applied alternating probe current and the measured resulting voltage a magnitude of the electrical impedance (31) of the xylem tissue (3), wherein an influence of the thermal state of the xylem tissue (3) on the magnitude of the electrical impedance (31) is accounted for; and
- determination means (19) configured to determine the relative change in the sap flow density in the vascular plant (1) by using a relation between the sap flow density and the magnitude of the electrical impedance (31) that an increased sap flow density results in an increased magnitude of the electrical impedance (31).

12. Measurement arrangement of claim 11, wherein the electrical impedance sensor (7, 10) is configured such that the first electrode (4) and the second electrode (5) are either spatially separated from each other or arranged adjacent to and partially isolated from each other.

13. Measurement arrangement of claim 12, wherein the first and second electrodes (4, 5) are two spatially separated needle-shaped electrodes, e.g. made of a non-corroding material, approximately 5 - 10 mm long, approximately 0.25 - 2 mm in diameter, respectively, and spatially separated from each other by approximately 1 - 10 centimeters (6) along a sprout axis of the vascular plant (1).

14. Measurement arrangement of claim 12, wherein the electrical impedance sensor (7, 10) is a bipolar measurement probe, wherein the first electrode (4) of an elongated form is sandwiched between the second electrode (4) and a single temperature sensor (9), wherein a first tip portion (4b) of the first electrode (4) projects compared to a second tip portion of the second electrode (5) and a third tip portion of the temperature sensor (9) in a direction of insertion (I) into the xylem tissue (3), wherein an interface between any pair of the first electrode (4), the second electrode (5), and the temperature sensor (9) is isolated from each other such that the projecting first tip portion (4b) is bare to be inserted into the xylem tissue (3) along with the bare second tip portion of the second electrode (5) and the bare third tip portion of the temperature sensor (9).

15. Measurement arrangement of any of claims 11 to 14, wherein at least one of the first and second electrodes (4, 5) has a tip (4c) showing dimensions that are small compared to a diameter of a stem of the vascular plant (1), e.g. such that a ratio of a diameter of the tip (4c) to the diameter of a stem of the vascular plant (1) is approximately 1/100, to allow for a minimally invasive insertion of the tip (4c) into the vascular plant (1).

**Patentansprüche**

1. Verfahren zum Bestimmen einer relativen Änderung einer Saftflussdichte in einer Gefäßpflanze (1), wobei das Verfahren die Schritte umfasst:

- Anordnen einer ersten Elektrode (4) und einer zweiten Elektrode (5) in der Gefäßpflanze (1) zur Bestimmung einer elektrischen Impedanz von Xylemgewebe (3) der Gefäßpflanze (1);
- entweder Anlegen einer Sondenwechselspannung (12) über die erste und die zweite Elektrode (4, 5) und Messen eines resultierenden Stroms (13), der von einer der ersten und der zweiten Elektrode (4, 5) zur anderen der ersten und der zweiten Elektrode (4, 5) fließt, oder Anlegen eines Sondenwechselstroms, der von einer der ersten und der zweiten Elektrode (4, 5) zur anderen der ersten und der zweiten Elektrode (4, 5) fließt, und Messen einer resultierenden Spannung über die erste und die zweite Elektrode (4, 5);

- Überwachen eines thermischen Zustands in dem Xylemgewebe (3), während entweder die Sondenwechselspannung (12) oder der Sondenwechselstrom angelegt wird;

- Berechnen einer Größe der elektrischen Impedanz (31) des Xylemgewebes (3) aus entweder der angelegten Sondenwechselspannung (12) und dem gemessenen resultierenden Strom (13) oder dem angelegten Sondenwechselstrom und der gemessenen resultierenden Spannung, wobei ein Einfluss des thermischen Zustands des Xylemgewebes (3) auf die Größe der elektrischen Impedanz (31) berücksichtigt wird; und

- Bestimmen der relativen Änderung der Saftflussdichte in der Gefäßpflanze (1) durch Verwenden einer Beziehung zwischen der Saftflussdichte und der Größe der elektrischen Impedanz (31), dass eine erhöhte Saftflussdichte zu einer erhöhten Größe der elektrischen Impedanz (31) führt.

2. Verfahren nach Anspruch 1, wobei zur Überwachung des thermischen Zustands im Xylemgewebe (3) eine Temperatur im Xylemgewebe (3) auf der Basis von Umgebungswetterbedingungen gemessen und/oder berechnet wird.

3. Verfahren nach Anspruch 1 oder 2, wobei entweder eine Sondenspannungsamplitude der angelegten Sondenwechselspannung (12) oder eine Sondenstromamplitude des angelegten Sondenwechselstroms so angelegt wird, dass entweder der gemessene resultierende Strom (13) oder die gemessene resultierende Spannung in einem im Wesentlichen linearen Bereich einer Strom-Spannungs-Beziehung entweder der angelegten Sondenwechselspannung (12) und des gemessenen resultierenden Stroms (13) oder des angelegten Sondenwechselstroms und der gemessenen resultierenden Spannung bleibt.

4. Verfahren nach Anspruch 3, wobei die elektrische Impedanz des Xylemgewebes (3) der Gefäßpflanze (1) über die Zeit bestimmt wird, wobei die relative Änderung der Saftstromdichte in der Gefäßpflanze (1) unter Verwendung einer Proportionalität bestimmt wird, dass eine zirkadiane Amplitude (37, 41) der täglichen Schwankungen der Größe der elektrischen Impedanz (31) proportional zur Saftstromdichte ist.

5. Verfahren nach Anspruch 3 oder Anspruch 4, wobei durch Bestimmung der elektrischen Impedanz des Xylemgewebes (3) der Gefäßpflanze (1) über mindestens zwei Tage ein quantitativer Indikator für einen Trockenstress der Gefäßpflanze (1) erzeugt wird, so dass eine Zunahme des Trockenstresses zu einer durchschnittlichen Zunahme der Größe der elektrischen Impedanz (31) über die mindestens zwei Tage führt, und die relative Änderung der Saftflussdichte in der Gefäßpflanze (1) unter Berücksichtigung zusätzlicher Informationen über eine artspezifische Reaktion des Spaltöffnungsapparates der Gefäßpflanze (1) auf den Trockenstress bestimmt wird.

6. Verfahren nach einem der Ansprüche 1 bis 5, wobei ein Einfluss des thermischen Zustands des Xylemgewebes (3) auf die Größe der elektrischen Impedanz (31) dadurch berücksichtigt wird, dass die Größe der elektrischen Impedanz (31) um eine Abhängigkeit der Größe der elektrischen Impedanz (31) von einer Temperatur des Xylemgewebes (3) korrigiert wird.

7. Verfahren nach Anspruch 1 oder Anspruch 2, wobei entweder eine Sondenspannungsamplitude der angelegten Sondenwechselspannung (12) oder eine Sondenstromamplitude des angelegten Sondenwechselstroms so angelegt wird, dass entweder der gemessene resultierende Strom (13) oder die gemessene resultierende Spannung in einem im Wesentlichen nichtlinearen Bereich einer Strom-Spannungs-Beziehung entweder der angelegten Sondenwechselspannung (12) und des gemessenen resultierenden Stroms (13) oder des angelegten Sondenwechselstroms und der gemessenen resultierenden Spannung liegt, wobei eine elektrische Leistung in das Xylemgewebe entweder durch eine vorbestimmte Sondenspannungsamplitude der angelegten Sondenwechselspannung (12) oder eine vorbestimmte Sondenstromamplitude des angelegten Sondenwechselstroms freigesetzt wird, was eine Erhöhung der Temperatur um vorzugsweise 5-10°C bewirkt.

8. Verfahren nach Anspruch 7, wobei sich bei einer vorbestimmten konstanten elektrischen Leistung im Xylemgewebe (3) nach Erreichen eines thermischen Gleichgewichts ein konstantes Übertemperaturprofil einstellt, wobei die Saftstromdichte eines umgebenden Xylemgewebes (3) eine Abkühlung des das Übertemperaturprofil aufweisenden Xylemgewebes (3) bewirkt und zu einer Erhöhung der Größe der elektrischen Impedanz (31) im Vergleich zu einer ohne Saftstrom ermittelten Größe der elektrischen Impedanz (31) führt.

9. Verfahren nach Anspruch 7 oder 8, wobei entweder die Größe der elektrischen Impedanz (31) bestimmt wird oder entweder die Sondenspannungsamplitude der angelegten Sondenwechselspannung (12) oder die Sondenstromamplitude des angelegten Sondenwechselstroms gesteuert wird, so dass die Größe der elektrischen Impedanz (31) im Wesentlichen konstant bleibt.

10. Softwareprogramm, das das Verfahren nach einem der vorhergehenden Ansprüche realisiert, wenn es auf einem System ausgeführt wird, das eine Messanordnung nach Anspruch 11 und einen Computer umfasst, wobei der Computer vorzugsweise ein verteiltes Rechensystem ist, von dem sich ein Teil in einem Cloud-Computersystem befindet.

11. Messanordnung zur Bestimmung einer relativen Änderung einer Saftflussdichte in einer Gefäßpflanze (1), wobei die Messanordnung umfasst:

- einen elektrischen Impedanzsensor (7, 10), der so konfiguriert ist, dass eine erste Elektrode (4) und eine zweite Elektrode (5) in der Gefäßpflanze (1) angeordnet sind, um eine elektrische Impedanz von Xylemgewebe (3) der Gefäßpflanze (1) zu bestimmen;
- eine Stromversorgungs- und elektrische Messeinrichtung (16), die so konfiguriert ist, dass sie entweder eine Sondenwechselspannung (12) über die erste und die zweite Elektrode (4, 5) anlegt und einen resultierenden Strom (13) misst, der von einer der ersten und der zweiten Elektrode (4, 5) zur anderen der ersten und der zweiten Elektrode (4, 5) fließt, oder dass sie einen Sondenwechselstrom anlegt, der von einer der ersten und der zweiten Elektrode (4, 5) zur anderen der ersten und der zweiten Elektrode (4, 5) fließt, und eine resultierende Spannung über die erste und die zweite Elektrode (4, 5) misst;
- eine thermische Überwachungseinrichtung (17), die so konfiguriert ist, dass sie einen thermischen Zustand im Xylemgewebe (3) überwacht, während entweder die Sonden-Wechselspannung (12) oder der Sonden-Wechselstrom angelegt wird;
- Berechnungsmittel (18), die so konfiguriert sind, dass sie entweder aus der angelegten Sondenwechselspannung (12) und dem gemessenen resultierenden Strom (13) oder dem angelegten Sondenwechselstrom und der gemessenen resultierenden Spannung eine Größe der elektrischen Impedanz (31) des Xylemgewebes (3) berechnen, wobei ein Einfluss des thermischen Zustands des Xylemgewebes (3) auf die Größe der elektrischen Impedanz (31) berücksichtigt wird; und
- eine Bestimmungseinrichtung (19), die so konfiguriert ist, dass sie die relative Änderung der Saftstromdichte in der Gefäßpflanze (1) unter Verwendung einer Beziehung zwischen der Saftstromdichte und der Größe der elektrischen Impedanz (31) bestimmt, wobei eine erhöhte Saftstromdichte zu einer erhöhten Größe der elektrischen Impedanz (31) führt.

12. Messanordnung nach Anspruch 11, wobei der elektrische Impedanzsensor (7, 10) so ausgebildet ist, dass die erste Elektrode (4) und die zweite Elektrode (5) entweder räumlich voneinander getrennt oder benachbart und teilweise isoliert voneinander angeordnet sind.

13. Messanordnung nach Anspruch 12, wobei die erste und die zweite Elektrode (4, 5) zwei räumlich voneinander getrennte nadelförmige Elektroden, z.B. aus einem nicht korrodierenden Material, mit einer Länge von ca. 5 -10 mm und einem Durchmesser von ca. 0,25 - 2 mm sind, die entlang einer Sprossachse der Gefäßpflanze (1) einen räumlichen Abstand von ca. 1 - 10 Zentimetern (6) zueinander aufweisen.

14. Messanordnung nach Anspruch 12, wobei der elektrische Impedanzsensor (7, 10) eine bipolare Messsonde ist, wobei die erste Elektrode (4) in länglicher Form zwischen der zweiten Elektrode (4) und einem einzigen Temperatursensor (9) angeordnet ist, wobei ein erster Spitzenabschnitt (4b) der ersten Elektrode (4) im Vergleich zu einem zweiten Spitzenabschnitt der zweiten Elektrode (5) und einem dritten Spitzenabschnitt des Temperatursensors (9) in einer Einführrichtung (I) in das Xylemgewebe (3) vorsteht, wobei eine Schnittstelle zwischen einem beliebigen Paar aus der ersten Elektrode (4), der zweiten Elektrode (5) und dem Temperatursensor (9) voneinander isoliert ist, so dass der vorstehende erste Spitzenabschnitt (4b) frei ist, um zusammen mit dem freien zweiten Spitzenabschnitt der zweiten Elektrode (5) und dem freien dritten Spitzenabschnitt des Temperatursensors (9) in das Xylemgewebe (3) eingeführt zu werden.

15. Messanordnung nach einem der Ansprüche 11 bis 14, wobei mindestens eine der ersten und zweiten Elektroden (4, 5) eine Spitze (4c) aufweist, die im Vergleich zu einem Durchmesser eines Stängels der Gefäßpflanze (1) kleine Abmessungen aufweist, z.B. so, dass ein Verhältnis eines Durchmessers der Spitze (4c) zu dem Durchmesser eines Stängels der Gefäßpflanze (1) ungefähr 1/100 beträgt, um ein minimalinvasives Einführen der Spitze (4c) in die Gefäßpflanze (1) zu ermöglichen.

**Revendications**

1. Procédé pour déterminer un changement relatif de la densité de flux de sève dans une plante vasculaire (1), le procédé comprenant les étapes consistant à :

   agencer une première électrode (4) et une deuxième électrode (5) dans la plante vasculaire (1) pour déterminer une impédance électrique de tissu xylémique (3) de la plante vasculaire (1) ;
   soit appliquer une tension de sonde alternative (12) sur la première et la deuxième électrode (4, 5) et mesurer un courant résultant (13) circulant de l'une de la première et la deuxième électrode (4, 5) à l'autre de la première et la deuxième électrode (4, 5), soit appliquer un courant de sonde alternatif circulant de l'une de la première et la deuxième électrode (4, 5) à l'autre de la première et la deuxième électrode (4, 5) et mesurer une tension résultante sur la première et la deuxième électrode (4, 5) ;
   surveiller un état thermique dans le tissu xylémique (3) tout en appliquant soit la tension de sonde alternative (12) soit le courant de sonde alternatif ; calculer, soit à partir de la tension de sonde alternative (12) appliquée et du courant résultant (13) mesuré, soit à partir du courant de sonde alternatif appliqué et de la tension résultante mesurée, une grandeur de l'impédance électrique (31) du tissu xylémique (3), une influence de l'état thermique du tissu xylémique (3) sur la grandeur de l'impédance électrique (31) étant prise en compte ; et
   déterminer le changement relatif de la densité de flux de sève dans la plante vasculaire (1) en utilisant une relation entre la densité de flux de sève et la grandeur de l'impédance électrique (31) selon laquelle une densité de flux de sève accrue entraîne une grandeur accrue de l'impédance électrique (31).

2. Procédé selon la revendication 1, dans lequel, pour surveiller l'état thermique dans le tissu xylémique (3), une température régnant dans le tissu xylémique (3) est mesurée et/ou calculée sur la base de conditions météorologiques ambiantes.

3. Procédé selon la revendication 1 ou la revendication 2, dans lequel soit une amplitude de tension de sonde de la tension de sonde alternative (12) appliquée, soit une amplitude de courant de sonde du courant de sonde alternatif appliqué est appliquée de telle sorte que soit le courant résultant (13) mesuré soit la tension résultante mesurée reste dans un régime sensiblement linéaire de relation courant-tension, soit de la tension de sonde alternative (12) appliquée et du courant résultant (13) mesuré, soit du courant de sonde alternatif appliqué et de la tension résultante mesurée.

4. Procédé selon la revendication 3, dans lequel l'impédance électrique du tissu xylémique (3) de la plante vasculaire (1) est déterminée dans le temps, le changement relatif de la densité de flux de sève dans la plante vasculaire (1) étant déterminé en utilisant une proportionnalité selon laquelle une amplitude circadienne (37, 41) de variations quotidiennes de la grandeur de l'impédance électrique (31) est proportionnelle à la densité de flux de sève.

5. Procédé selon la revendication 3 ou la revendication 4, dans lequel, en déterminant l'impédance électrique du tissu xylémique (3) de la plante vasculaire (1) sur au moins deux jours, un indicateur quantitatif d'un stress hydrique de la plante vasculaire (1) est généré de telle sorte qu'une augmentation du stress hydrique entraîne une augmentation de la moyenne de la grandeur de l'impédance électrique (31) sur les au moins deux jours, et le changement relatif de la densité de flux de sève dans la plante vasculaire (1) est déterminé en prenant en compte des informations supplémentaires concernant une réaction de l'appareil stomatique de la plante vasculaire (1) au stress hydrique spécifique à l'espèce.

6. Procédé selon l'une quelconque des revendications 1 à 5, dans lequel une influence de l'état thermique du tissu xylémique (3) sur la grandeur de l'impédance électrique (31) est prise en compte du fait que la grandeur de l'impédance électrique (31) est corrigée pour une dépendance entre la grandeur de l'impédance électrique (31) et une température du tissu xylémique (3).

7. Procédé selon la revendication 1 ou la revendication 2, dans lequel soit une amplitude de tension de sonde de la tension de sonde alternative (12) appliquée soit une amplitude de courant de sonde du courant de sonde alternatif appliqué est appliquée de telle sorte que soit le courant résultant (13) mesuré, soit la tension résultante mesurée se trouve dans un régime sensiblement non linéaire de relation courant-tension, soit de la tension de sonde alternative (12) appliquée et du courant résultant (13) mesuré, soit du courant de sonde alternatif appliqué et de la tension résultante mesurée, une puissance électrique étant libérée dans le tissu xylémique soit par une amplitude de tension de sonde prédéterminée de la tension de sonde alternative (12) appliquée soit par une amplitude de courant de sonde prédéterminée du courant de sonde alternatif appliqué, provoquant une augmentation de la température de préférence comprise entre 5 et 10 °C.

8. Procédé selon la revendication 7, dans lequel, à une puissance électrique constante prédéterminée dans le tissu xylémique (3), un profil de surtempérature constante est mis en place après qu'un équilibre thermique est atteint, la densité de flux de sève d'un tissu xylémique (3) environnant réalisant un refroidissement du tissu xylémique (3) qui présente le profil de surtempérature et entraînant une augmentation de la grandeur de l'impédance électrique (31) par rapport à une grandeur de l'impédance électrique (31) déterminée sans flux de sève.

9. Procédé selon la revendication 7 ou la revendication 8, dans lequel soit la grandeur de l'impédance électrique (31) est déterminée, soit l'amplitude de tension de sonde de la tension de sonde alternative (12) appliquée ou l'amplitude de courant de sonde du courant de sonde alternatif appliqué est commandée, de telle sorte que la grandeur de l'impédance électrique (31) reste sensiblement constante.

10. Programme logiciel réalisant le procédé selon l'une quelconque des revendications précédentes quand il est exécuté sur un système comprenant un agencement de mesure selon la revendication 11 et un ordinateur, l'ordinateur étant de préférence un système informatique distribué dont une partie est située dans un système informatique en nuage.

11. Agencement de mesure pour déterminer un changement relatif de la densité de flux de sève dans une plante vasculaire (1), l'agencement de mesure comprenant :

un capteur d'impédance électrique (7, 10), conçu de telle sorte qu'une première électrode (4) et une deuxième électrode (5) sont agencées dans la plante vasculaire (1) pour déterminer une impédance électrique de tissu xylémique (3) de la plante vasculaire (1) ;
une alimentation électrique et des moyens de mesure électrique (16) conçus pour soit appliquer une tension de sonde alternative (12) sur la première et la deuxième électrode (4, 5) et mesurer un courant résultant (13) circulant de l'une de la première et la deuxième électrode (4, 5) à l'autre de la première et la deuxième électrode (4, 5), soit appliquer un courant de sonde alternatif circulant de l'une de la première et la deuxième électrode (4, 5) à l'autre de la première et la deuxième électrode (4, 5) et mesurer une tension résultante sur la première et la deuxième électrode (4, 5) ;
des moyens de surveillance thermique (17) conçus pour surveiller un état thermique dans le tissu xylémique (3) tout en appliquant soit la tension de sonde alternative (12) soit le courant de sonde alternatif ;
des moyens de calcul (18) conçus pour calculer, soit à partir de la tension de sonde alternative (12) appliquée et du courant résultant (13) mesuré, soit à partir du courant de sonde alternatif appliqué et de la tension résultante mesurée, une grandeur de l'impédance électrique (31) du tissu xylémique (3), une influence de l'état thermique du tissu xylémique (3) sur la grandeur de l'impédance électrique (31) étant prise en compte ; et
des moyens de détermination (19) conçus pour déterminer le changement relatif de la densité de flux de sève dans la plante vasculaire (1) en utilisant une relation entre la densité de flux de sève et la grandeur de l'impédance électrique (31) selon laquelle une densité de flux de sève accrue entraîne une grandeur accrue de l'impédance électrique (31).

12. Agencement de mesure selon la revendication 11, dans lequel le capteur d'impédance électrique (7, 10) est conçu de telle sorte que la première électrode (4) et la deuxième électrode (5) sont soit séparées l'une de l'autre dans l'espace, soit agencées adjacentes l'une à l'autre et en partie isolées l'une de l'autre.

13. Agencement de mesure selon la revendication 12, dans lequel la première et la deuxième électrodes (4, 5) sont deux électrodes en forme d'aiguille séparées dans l'espace, par exemple fabriquées en matériau résistant à la corrosion, respectivement d'une longueur d'environ 5 à 10 mm et d'un diamètre d'environ 0,25 à 2 mm , et séparées l'une de l'autre dans l'espace par environ 1 à 10 centimètres (6) le long d'un axe de rejet de la plante vasculaire (1).

14. Agencement de mesure selon la revendication 12, dans lequel le capteur d'impédance électrique (7, 10) est une sonde de mesure bipolaire, la première électrode (4) de forme allongée étant prise en sandwich entre la deuxième électrode (4) et un unique capteur de température (9), une première partie formant pointe (4b) de la première électrode (4) faisant saillie par rapport à une deuxième partie formant pointe de la deuxième électrode (5) et une troisième partie formant pointe du capteur de température (9) dans une direction d'insertion (I) dans le tissu xylémique (3), une interface entre toute paire formée par la première électrode (4), la deuxième électrode (5) et le capteur de température (9) étant isolée de l'autre de telle sorte que la première partie formant pointe (4b) qui fait saillie est nue pour être insérée dans le tissu xylémique (3) avec la deuxième partie formant pointe nue de la deuxième électrode (5) et la troisième partie formant pointe nue du capteur de température (9).

**15.** Agencement de mesure selon l'une quelconque des revendications 11 à 14, dans lequel au moins une de la première et la deuxième électrode (4, 5) présente une pointe (4c) présentant des dimensions qui sont petites par rapport à un diamètre d'une tige de la plante vasculaire (1), par exemple de telle sorte qu'un rapport entre un diamètre de la pointe (4c) et le diamètre d'une tige de la plante vasculaire (1) est d'environ 1/100, afin de permettre une insertion mini-invasive de la pointe (4c) dans la plante vasculaire (1).

# Fig. 1

# Fig. 2

# Fig. 3

# Fig. 4

## Fig. 5

## Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017123010 A1
- KR 101240398 B1
- EP 3104135 A1